# EUROPÄISCHE PATENTANMELDUNG

(11) **EP 2 378 277 A2**
(43) Veröffentlichungstag der Anmeldung: **19.10.2011**
(21) Anmeldenummer: 11003162.2
(22) Anmeldetag: 14.04.2011
(51) Int. Cl.: G01N 21/85, G01N 33/28, G01N 21/33

(54) **Sensor und Verfahren zur Online-Überwachung der Säurezahl eines Hydraulikfluids in einem Hydraulikstystem in einem Luftfahrzeug**

(30) Priorität: 15.04.2010 DE 102010015083
(71) Anmelder: EADS Deutschland GmbH, 85521 Ottobrunn (DE)
(72) Erfinder: Müller, Gerhard, Dr., 85567 Grafing (DE); Sumit, Paul, 81677 München (DE); Helwig, Andreas, Dr., 80469 München (DE)
(74) Vertreter: Schäfer, Matthias W.

(57) **Zusammenfassung**

Die Erfindung betrifft einen Sensor (301) zur Online-Überwachung der Säurezahl eines Hydraulikfluids in einem Hydrauliksystem eines Luftfahrzeuges, umfassend:
- eine UV-Emittereinrichtung (315) zum Emittieren von UV-Licht,
- eine UV-Detektoreinrichtung (317) zum Detektieren von UV-Licht,
- eine Aufnahmeeinrichtung (303) zum Aufnehmen des Hydraulikfluids,
- eine Einkoppeleinrichtung (307; 313) zum Einkoppeln von mittels der UV-Emittereinrichtung (315) emittierten UV-Licht in die Aufnahmeeinrichtung (303), um das aufgenommene Hydraulikfluid optisch anzuregen, und
- eine Auskoppeleinrichtung (307; 313) zum Auskoppeln von mittels des optisch angeregten Hydraulikfluids emittierten Licht in die UV-Detektoreinrichtung (317).

Die Erfindung betrifft ferner ein entsprechendes Verfahren.

## Beschreibung

Die Erfindung betrifft einen Sensor und ein Verfahren zur Online-Überwachung der Säurezahl eines Hydraulikfluids in einem Hydrauliksystem in einem Luftfahrzeug.

### Hintergrund der Erfindung

Hydraulische Fluide für Hydrauliksysteme eines Luftfahrzeuges sind in der Regel hydroskopisch. Durch die Aufnahme von Wasser können allerdings in dem Fluid chemische Reaktionen ausgelöst werden, in welchen das Hydrauliksystem schädigende Produkte gebildet werden. Beispielsweise können korrosive Produkte gebildet werden, welche insbesondere die Dichtungen und die Oberflächen der Hydraulikschläuche des Hydrauliksystems angreifen. Hieraus resultiert eine kontinuierliche Verschlechterung der Leistungsfähigkeit des Hydrauliksystems bis hin zu einem Totalausfall des Hydrauliksystems. Das wiederum beeinträchtigt in einem erheblichen Maße eine Flugsicherheit des Luftfahrzeugs und kann sogar zu einem Absturz des Luftfahrzeugs führen.

Da Hydrauliksysteme für Luftfahrzeuge üblicherweise einen geschlossenen Hydraulikkreislauf bilden, ist es besonders schwer, korrodierte oder beschädigte Hydraulikelemente zu erfassen. Ein Austausch solcher Hydraulikelemente kostet Zeit und Geld und ist sehr aufwendig.

Es ist daher wünschenswert, den Zustand des hydraulischen Fluids zu bestimmen, insbesondere eine Säurezahl des Fluids, um frühzeitig Beschädigungen im Hydrauliksystem zu verhindern.

Es ist bekannt, eine Probe des Fluids aus dem Hydrauliksystem zu entnehmen und diese Probe in einem Labor zu untersuchen. Nachteilig hieran ist aber, dass dieses Untersuchungsverfahren zeitaufwendig ist, da zunächst die Probe entnommen werden, in das Labor geschickt und dort untersucht werden muss.

Deshalb wurde in der DE 10 2006 060 138 B4 vorgeschlagen, einen Online-Sensor in das Hydrauliksystem zu integrieren. Der bekannte Online-Sensor weist eine Aufnahmeeinheit auf umfassend einen Probenbehälter zur Aufnahme einer Probe eines zu überwachenden Hydraulikfluids. Der Probenbehälter besteht aus einem dünnen scheibenförmigen Aluminiumbehälter mit einem Volumen von wenigen Kubikzentimetern. Der Aluminiumbehälter weist an seinen Stirnseiten Sichtfenster aus Saphirglas und den Sichtfenstern gegenüberliegend angeordnet jeweils einen IR-Emitter und einen IR-Detektor auf. Das sich in dem Probenbehälter befindende Hydraulikfluid wird so mit IR-Licht bestrahlt, um das IR-Absorptionsspektrum des Hydraulikfluids zu messen.

Nachteilig hieran ist aber, dass aufgrund des kleinen Volumens des Probenbehälters dieser bei einer direkten Integration in einen Hydraulikschlauch wie ein Verschluss wirkt und so einen Hydraulikfluiddurchfluss behindert. Hierbei kann der Probenbehälter aber auch nicht beliebig vergrößert werden, da sonst die Intensität des IR-Absorptionsspektrums stark abnehmen würde.

### Zusammenfassung der Erfindung

Es ist daher die der Erfindung zugrunde liegenden Aufgabe, einen Sensor und ein Verfahren bereitzustellen, welche die obigen Nachteile überwinden und eine Online-Überwachung eines Hydraulikfluids in einem Hydrauliksystem eines Luftfahrzeugs ermöglichen, ohne dass ein Hydraulikfluiddurchfluss behindert wird.

Die Aufgabe wird mittels eines Sensors nach Anspruch 1 und mittels eines Verfahrens nach Anspruch 10 gelöst. Vorteilhafte Ausgestaltungen der Erfindung sind Gegenstand von abhängigen Unteransprüchen.

Die Erfindung umfasst den Gedanken einen Sensor zur Online-Überwachung der Säurezahl eines Hydraulikflluids in einem Hydrauliksystem eines Luftfahrzeuges bereitzustellen, umfassend:
- eine UV-Emittereinrichtung zum Emittieren von UV-Licht,
- eine UV-Detektoreinrichtung zum Detektieren von UV-Licht,
- eine Aufnahmeeinrichtung zum Aufnehmen des Hydraulikfluids,
- eine Einkoppeleinrichtung zum Einkoppeln von mittels der UV-Emittereinrichtung emittierten UV-Licht in die Aufnahmeeinrichtung, um das aufgenommene Hydraulikfluid optisch anzuregen, und
- eine Auskoppeleinrichtung zum Auskoppeln von mittels des optisch angeregeten Hydraulikfluids emittierten Licht in die UV-Detektoreinrichtung.

Die Erfindung umfasst weiterhin den Gedanken, ein Verfahren zur Online-Überwachung der Säurezahl eines Hydraulikfluids in einem Hydrauliksystem eines Luftfahrzeuges bereitzustellen, umfassend die folgenden Schritte:
- Emittieren von UV-Licht mittels einer UV-Emittereinrichtung,
- Einkoppeln des emittierten UV-Lichts mittels einer Einkoppeleinrichtung in eine Aufnahmeeinrichtung zum Aufnehmen des Hydraulikfluids, so dass das aufgenommene Hydraulikfluid optisch angeregt wird,
- Auskoppeln von mittels des optisch angeregten Hydraulikfluids emittierten Licht mittels einer Auskoppeleinrichtung in eine UV-Detektoreinrichtung.

Erfindungsgemäß wird das Hydraulikfluid mittels UV-Licht, insbesondere mit einer Wellenlänge von kleiner als 400nm, bevorzugterweise mit einer Wellenlänge von kleiner als 300nm, beispielsweise mit einer Wellenlänge von 275nm, optisch angeregt und ein Lumineszenzspektrum des optisch angeregten Hydraulikfluids gemessen. Hierbei zeigt sich, dass das Lumineszenzspektrum abhängig von der Säurezahl des Hydraulikfluids ist. Die Säurezahl ist eine chemische Größe zur Charakterisierung von sauren Bestandteilen in Stoffgemischen. Mit ihr werden alle sauren Funktionen, die durch Kalilauge neutralisierbar sind, erfasst. Die Säurezahl gibt die Masse Kaliumhydroxid in Milligramm an, die zur Neutralisation von 1 g der zu untersuchenden Probe erforderlich ist. Die Säurezahl wird auch als Neutralisationszahl bezeichnet.

Die Säurezahl des Hydraulikfluids ändert sich also, wenn sich der Anteil an sauren Bestandteilen des Hydraulikfluids ändert. Eine solche Änderung des Anteils an sauren Bestandteilen wird insbesondere dadurch bewirkt, dass das Hydraulikfluid Wasser aufnimmt, wodurch chemische Reaktionen in dem Hydraulikfluid ausgelöst werden, insbesondere, wenn das Hydraulikfluid erwärmt wird. In solchen chemischen Reaktionen werden unter anderem korrosive Stoffe gebildet, so dass der Anteil an sauren Bestandteilen in dem Hydraulikfluid zunimmt und damit auch die Säurezahl.

Die Erfindung bietet hier insbesondere den Vorteil, dass eine Online-Überwachung des Zustandes des Hydraulikfluids möglich ist. Online bedeutet hier, dass das Hydraulikfluid in Echtzeit überwacht und untersucht werden kann. Es ist nicht erforderlich, eine Probe des Hydraulikfluids aus dem Hydrauliksystem zu entnehmen, um diese dann zeitaufwendig und kostenintensiv in ein Speziallabor zu schicken. Eine solches Untersuchungsverfahren kann zur Abgrenzung auch als ein Offline-Überwachungsverfahren bezeichnet werden. Insbesondere kann während eines Betriebes des Luftfahrzeugs die Säurezahl überwacht und gemessen werden. Eine kontinuierliche Erfassung des Zustandes des Hydraulikfluids ist somit ermöglicht. Folglich kann frühzeitig, also insbesondere noch bevor die korrosiven Anteile erhebliche Schäden im Hydrauliksystem bewirken, das Hydraulikfluid ausgetauscht oder wiederaufbereitet werden. Hierdurch wird in vorteilhafter Weise eine Flugsicherheit des Luftfahrzeugs erhöht. Gleichzeitig werden mittels der Erfindung Kosten eingespart.

Im Sinne der Erfindung kann ein Hydraulikfluid eine Hydraulikflüssigkeit oder ein Hydraulikgas sein. Insbesondere ist das Hydraulikfluid geeignet, spezielle Flugsicherheitsbestimmungen zu erfüllen, beispielsweise bezüglich einer Feuersicherheit. Das Hydraulikfluid kann bevorzugterweise Phosphat-Ester-Moleküle umfassen. Wenn dann Wasser von dem Phosphat-Ester-Moleküle umfassende Hydraulikfluid absorbiert wird, desintegrieren die Phosphat-Ester-Moleküle entlang insbesondere eines der folgenden Reaktionswege: Hydrolyse, Pyrolyse und Oxidation. Die hieraus entstehenden Produkte umfassen Alkohole und Phosphorsäuren. Die Desintegration läuft verstärkt ab, wenn das Hydraulikfluid erwärmt wird. Das ist in der Regel bei einem Flugbetrieb des Luftfahrzeugs der Fall. Mittels der Erfindung kann über die Bestimmung der Säurezahl der Anteil an Phosphorsäuren erfasst werden, so dass, falls dieserAnteil größer als ein vorbestimmter Wert ist, das Hydraulikfluid ausgetauscht oder wieder aufbereitet werden kann.

In einer beispielhaften Ausführungsform der Erfindung können die Einkoppeleinrichtung und/oder die Auskoppeleinrichtung ein Fenster aus einem für UV-Licht transparenten Material aufweisen. Beispielsweise kann das Material Saphierglas sein. Hierdurch wird insbesondere eine geringe Streuung bei einem Strahlendurchgang des Lichts durch die Fenster bewirkt. Weiterhin ist ein Fenster aus Saphierglas besonders mechanisch stabil und chemisch inert, so dass ein solches Fenster auch den Bedingungen in einem Hydrauliksystem widerstehen können, insbesondere Drücken von bis zu 400 bar und/oder Temperaturen von bis zu 250° C. Nach einer weiteren bevorzugten Ausgestaltung der Erfindung kann vorgesehen sein, dass die Auskoppeleinrichtung und die Einkoppeleinrichtung ein gemeinsames Fenster aus einem für UV-Licht transparenten Material, insbesondere ein Fenster aus Saphierglas, umfassen, wobei durch das gemeinsame Fenster sowohl das emittierte UV-Licht in die Aufnahmeeinrichtung eingekoppelt als auch das Lumineszenzlicht aus der Aufnahmeeinrichtung in die UV-Detektoreinrichtung ausgekoppelt werden.

Nach einer weiteren bevorzugten Ausführungsform der Erfindung weisen die Einkoppeleinrichtung und/oder die Auskoppeleinrichtung eine optische Faser auf. Hierdurch kann in vorteilhafter Weise das Licht gezielt in die bzw. aus der Aufnahmeeinrichtung ein- bzw. ausgekoppelt werden, so dass eine Intensität des Lumineszenzspektrums gesteigert werden kann. Damit erhöht sich insbesondere die Nachweisempfindlichkeit bezüglich korrosiver Bestandteile. Beispielsweise können die Einkoppeleinrichtung und die Auskoppeleinrichtung eine gemeinsame optische Faser aufweisen. Eine solche gemeinsame optische Faser weist insbesondere eine Y-Form auf, so dass die optische Faser als ein Strahlteiler wirkt. Durch den einen Arm des Ypsilons wird UV-Licht in die Aufnahmeeinrichtung eingekoppelt und durch den anderen Arm wird dann das Lumineszenzlicht zu der UV-Detektoreinrichtung geführt und in diese eingekoppelt.

Gemäß einer anderen beispielhaften Ausgestaltung der Erfindung sind die Einkoppeleinrichtung und die Auskoppeleinrichtung derart gegenüberliegend angeordnet, dass ein optischer Strahlengang zwischen der Einkoppeleinrichtung und der Auskoppeleinrichtung gebildet ist. Beispielsweise kann hier ein Hydraulikschlauch eines Hydrauliksystems zwei gegenüberliegende Fenster, insbesondere Fenster aus Saphierglas, aufweisen, wobei die Einkoppeleinrichtung das UV-Licht durch das eine Fenster in den Hydraulikschlauch einkoppelt und das Lumineszenzlicht mittels des anderen Fensters aus dem Hydraulikschlauch ausgekoppelt wird. In diesem Ausführungsbeispiel ist die Aufnahmeeinrichtung insofern integral mit dem Hydraulikschlauch gebildet. Allerdings soll sich die allgemeine Idee, dass die Aufnahmeeinrichtung integral mit einem Hydraulikschlauch eines Hydrauliksystems gebildet ist, nicht nur auf dieses spezielle Ausführungsbeispiel beschränken. Allgemein gesagt ist nach einer besonders bevorzugten Ausgestaltung der Erfindung die Aufnahmeeinrichtung integral mit einem Hydraulikschlauch eines Hydrauliksystems, insbesondere eines Luftfahrzeugs, gebildet.

Nach einer weiteren bevorzugten Ausführungsform der Erfindung kann der Hydraulikschlauch aus einem für UV-Licht transparenten Material gebildet sein. Es kann aber beispielsweise auch nur vorgesehen sein, dass ein Abschnitt des Hydraulikschlauchs aus einem solchen Material gebildet sind, insbesondere sind zwei gegenüberliegende Abschnitte aus einem solchen Material gebildet. Hierbei kann der Hydraulikschlauch bzw. der oder die beiden Abschnitte das oder die Fenster aus einem für UV-Licht transparenten Material ersetzen oder ergänzen. Dadurch ist ein besonders kompakter und integraler Aufbau des Sensors in einem Hydrauliksystem ermöglicht. Die Einkoppeleinrichtung und/oder die Auskoppeleinrichtung sind insofern zumindest teilweise in den Hydraulikschlauch bzw. in das Hydrauliksystem integriert.

Gemäß einer anderen beispielhaften Ausgestaltung der Erfindung weist die Detektoreinrichtung eine Auswerteeinrichtung zum Auswerten des detektierten Lichts auf. Hierdurch kann sofort das Lumineszenzspektrum ausgewertet und somit die Säurezahl bestimmt werden. Die Auswerteeinrichtung kann insbesondere einen Netzwerkanschluss aufweisen, mittels welchen die Auswerteeinrichtung an ein Netzwerk, beispielsweise ein Bordnetzwerk eines Luftfahrzeugs, angeschlossen werden kann. Über das Netzwerk kann dann beispielsweise die Säurezahl in das Cockpit übermittelt werden, so dass der Pilot jederzeit Zugriff auf diese Information hat. Es kann insbesondere auch vorgesehen sein, dass die Säurezahl an eine Bodenstation gesendet wird, so dass insbesondere der Betreiber des Luftfahrzeugs Zugriff auf diese Information hat.

Nach einer weiteren bevorzugten Ausführungsform der Erfindung weist die Auswerteeinrichtung eine Speichereinrichtung auf, in welcher eine Korrelation zwischen einer Wellenlänge und einer Säurezahl gespeichert ist. Eine solche Korrelation wird bevorzugterweise mittels Laborversuche gebildet. Beispielsweise indem ein Hydraulikfluid gezielt verunreinigt wird und die Säurezahl mittels eines dem Fachmann bekannten Standardverfahrens bestimmt wird, insbesondere mittels eines Titrationsverfahrens. Dann wird das gezielt verunreinigte Hydraulikfluid mit UV-Licht bestrahlt und das entsprechende Lumineszenzspektrum gemessen. Dieses gemessene Spektrum wird dann der mittels des Standardverfahrens bestimmten Säurezahl zugeordnet. Somit kann also eine Korrelation zwischen einer Wellenlänge und einer Säurezahl gebildet werden. Insbesondere ist so eine Korrelation zwischen einem Lumineszenzspektrum und einer Säurezahl gebildet. Da sich übliche für Luftfahrzeuge geeignete Hydraulikfluide in ihren Bestandteilen unterscheiden können, kann für jedes Hydraulikfluid eine spezifische Kennlinie gebildet werden, welche in der erfindungsgemäßen Speichereinrichtung gespeichert werden kann. So kann je nach verwendetem Hydraulikfluid die entsprechende Kennlinie zur Auswertung des Lumineszenzspektrums verwendet werden. Nach einer weiteren bevorzugten Ausgestaltung der Erfindung kann die Speichereinrichtung austauschbar gebildet sein. So ist es in vorteilhafter Weise ermöglicht, schnell und einfach bei einem Austausch des Hydraulikfluids den Sensor entsprechend anzupassen, indem eine neue Speichereinrichtung mit der darin abgespeicherten entsprechenden Kennlinie in den Sensor eingesetzt wird.

Nach einer weiteren beispielhaften Ausführungsform der Erfindung umfasst die UV-Emittereinrichtung eine LED, insbesondere eine UV-LED. In noch einer weiteren beispielhaften Ausführungsform der Erfindung umfasst die UV-Detektoreinrichtung einen Photodetektor. Die UV-Detektoreinrichtung umfasst insbesondere ein Spektrometer. Beispielsweise kann an dem Spektrometer noch ein Verstärker zum Verstärken von dem Lumineszenzlicht zugeordneten Messsignalen angeschlossen sein.

Nach einer anderen bevorzugten Ausgestaltung der Erfindung kann vorgesehen sein, dass die Aufnahmeeinrichtung in dem Hydrauliksystem angeordnet ist. Im Sinne der Erfindung umfasst das Hydrauliksystem Hydraulikschläuche, Hydraulikpumpen, Hydraulikfluide und Hydraulikdichtungen. Insbesondere kann die Aufnahmeeinrichtung integral mit dem Hydrauliksystem gebildet sein, beispielsweise mit einem Hydraulikschlauch. Insbesondere kann vorgesehen sein, das die UV-Emittereinrichtung und die UV-Detektoreinrichtung außerhalb des Hydrauliksystems angeordnet sind, insbesondere außerhalb eines Hydraulikschlauchs. Hierdurch wird in vorteilhafter Weise vermieden, dass die UV-Emittereinrichtung und die UV-Detektoreinrichtung nicht den Einflüssen des Hydrauliksystems unterliegen. Beispielsweise kann ein Druck in dem Hydrauliksystem bis zu 400bar und eine Temperatur bis zu 250° C betragen. Folglich wird dadurch insbesondere eine Lebensdauer der UV-Emittereinrichtung und der UV-Detektoreinrichtung erhöht.

Gemäß einer beispielhaften Ausführungsform des Verfahrens kann vorgesehen sein, die Säurezahl in vorbestimmten Zeitabständen zu bestimmen. Somit ist es ermöglicht, einen zeitlichen Verlauf der Säurezahl zu erfassen. Insbesondere kann dadurch eine Degradierung des Hydraulikfluids erfasst werden. Beispielsweise kann vorgesehen sein, den zeitlichen Verlauf der Säurezahl zeitlich in die Zukunft zu extrapolieren, so dass abgeschätzt werden kann, wann ein Austausch des Hydraulikfluids notwendig sein wird. Mittels der Extrapolation ist es insbesondere ermöglicht, weitere Wartungsarbeiten mit dem Austausch des Hydraulikfluids zu koordinieren, so dass eine Wartungszeit, in der das Luftfahrzeug nicht im Betrieb ist, kurz ist, wodurch in effizienter Weise weitere Kosten eingespart werden können.

### Beschreibung bevorzugter Ausführungsbeispiele

Weitere bevorzugte Ausführungsbeispiele der Erfindung werden anhand der Figuren beschrieben. Hierbei zeigen
- Fig. 1: eine schematische Darstellung einer Desintegration eines Phosphat-Ester-Moleküls,
- Fig. 2: Lumineszenzspektren von Proben eines Hydraulikfluids mit unterschiedlichen Säurezahlen,
- Fig. 3: einen Sensor mit einer in einem Hydraulikschlauch integrierten Aufnahmeeinrichtung und
- Fig. 4: einen weiteren Sensor mit einer in einem Hydraulikschlauch integrierten Aufnahmeeinrichtung.

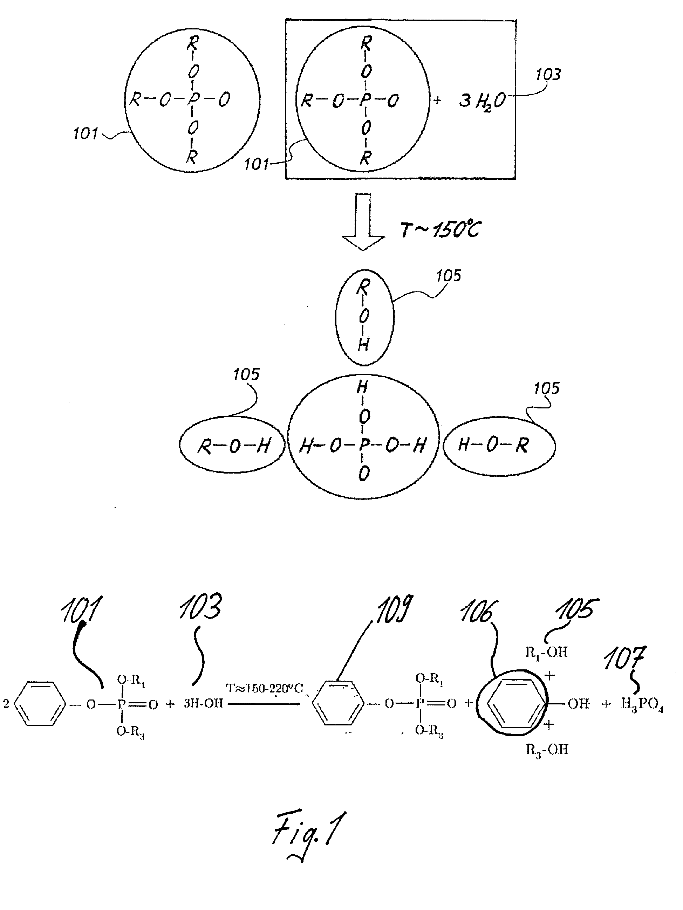

Fig. 1 zeigt eine schematische Darstellung einer Desintegration eines Phosphat-Ester-Moleküls 101, welches von einem Hydraulikfluid (nicht gezeigt) umfasst ist, in Anwesenheit von drei Wassermolekülen 103. Bei einer Temperatur von ca. 150° C bis 220° C läuft die Desintegration verstärkt ab. Das Phosphat-Ester-Molekül 101 spaltet mittels Hydrolyse solange Alkoholgruppen 105 ab, bis keine Alkoholgruppen 105 am Phosphat-Ester-Moleküls 101 mehr vorhanden sind und nur noch die Basisphosphatgruppe 106 des Phosphat-Ester-Moleküls 101 übrig ist, welches in eine Phosphorsäure 107 umgesetzt wird. Das Hydraulikfluid umfasst nach der Hydrolyse nicht umgewandelte Phosphat-Ester-Moleküle 109, zumindest teilweise desintegrierte Phosphat-Ester-Moleküle 111, freie Alkoholgruppen 105 und Phosphorsäure 107.

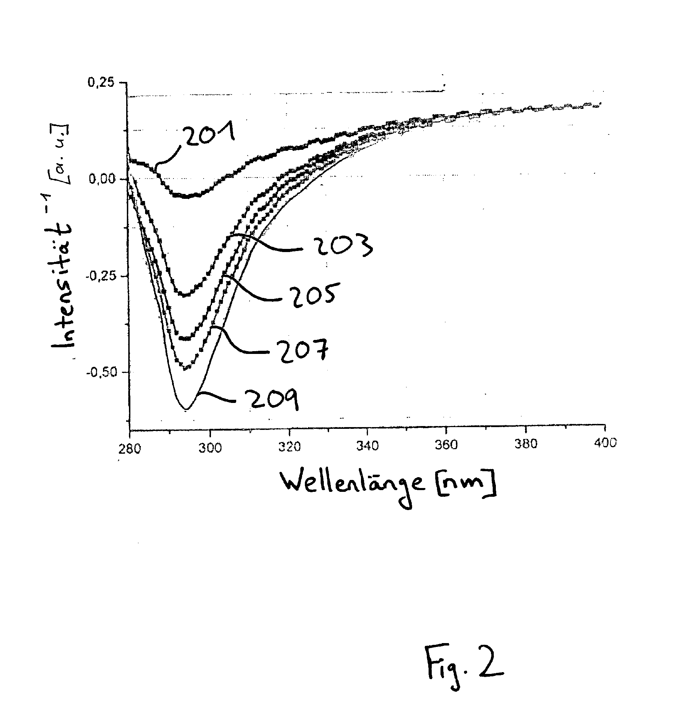

Fig. 2 zeigt fünf Lumineszenzspektren 201, 203, 205, 207 und 209 von jeweils einer - Probe eines Hydraulikfluids mit unterschiedlichen Säurezahlen. Aufgetragen ist die Intensität in willkürlichen Einheiten über die Wellenlänge in Nanometern. Das Hydraulikfluid umfasst Phenylgruppen. Ein solches Phenylgruppen umfassende kommerziell erhältliche Hydraulikfluid ist beispielsweise unter dem Markennamen "Skydrol" bekannt und erhältlich. Vier Proben wurde jeweils mit einer bestimmten unterschiedlichen Menge an Wasser verunreinigt. Anschließend wurden die Proben erhitzt, um die Hydrolyse zu starten. Es wurde dann die Säurezahl des Hydraulikfluids in den fünf Proben mittels eines Titrationsverfahrens bestimmt. Dann wurden die Proben in eine Messzelle mit optischen Saphierglasfenstern angeordnet und durch ein erstes Saphierglasfenster mit UV-Licht mit einer Wellenlänge von ca. 275nm bestrahlt. Das UV-Licht wurde mittels einer UV-LED erzeugt und von dieser emittiert. Durch ein zweites Saphierglasfenster wurde das Lumineszenzspektrum mittels eines Spektrometers gemessen, wobei die Messsignale mittels eines Verstärkers verstärkt wurden. Hierbei wurde kein Filter verwendet. Die gemessenen Lumineszenzspektren zeigen einen Scheitelpunkt bei ca. 295nm. Die jeweilige Intensität des Scheitelpunkts ist um so größer je größer die Säurezahl des Hydraulikfluids ist. Hierbei ist das Lumineszenzspektrum 201 der nicht-verunreinigten Probe, das heißt das Hydraulikfluid ohne gezielte Wasserverunreinigung, zugeordnet, wobei dieses Hydraulikfluid bereits eine Säurezahl von 0,14 aufweist. Das Lumineszenzspektrum 203 ist dem Hydraulikfluid mit einer Säurezahl von 0,3105, das Lumineszenzspektrum 205 ist dem Hydraulikfluid mit einer Säurezahl von 0,67, das Lumineszenzspektrum 207 ist dem Hydraulikfluid mit einer Säurezahl von 1,2 und das Lumineszenzspektrum 209 ist dem Hydraulikfluid mit einer Säurezahl von 2,1 zugeordnet.

Bei der Hydrolyse des Phenylgruppen umfassende Hydraulikfluid bilden sich freie Phenolalkohole, wobei einer größere Anzahl an freien Phenolalkoholen mit einer größeren Anzahl an Phosphorsäuren aufgrund der obigen Ausführungen, insbesondere bezüglich Fig. 1, korrelieren. Je verunreinigter das Hydraulikfluid ist, desto mehr freie Phenolalkohole und Phosphorsäuren bilden sich. In der Folge steigt die Säurezahl.

Phenolalkohole, welche noch an den Phosphat-Ester-Molekülen gebunden sind, "quenchen" das eingekoppelte UV-Licht optisch. Das heisst, sie absorbieren die Energie des UV-Lichts, ohne dass die absorbierte Energie in Lumineszenzlicht umgewandelt wird. Dieses optische Quenching hat seine Ursache insbesondere in der starken Elektronenverbindung zu den Phosphatgruppen. Wenn die anfangs gebundenen Phenolalkohole sich dann lösen, verringert sich auch das optische Quenching. Die Intensität des Lumineszenzlichts nimmt insofern zu.

Zusammenfassend zeigen diese Versuche, dass eine Säurezahl mit der Intensität eines Lumineszenzlichts eines mittels UV-Licht optisch angeregten Hydraulikfluids korreliert.

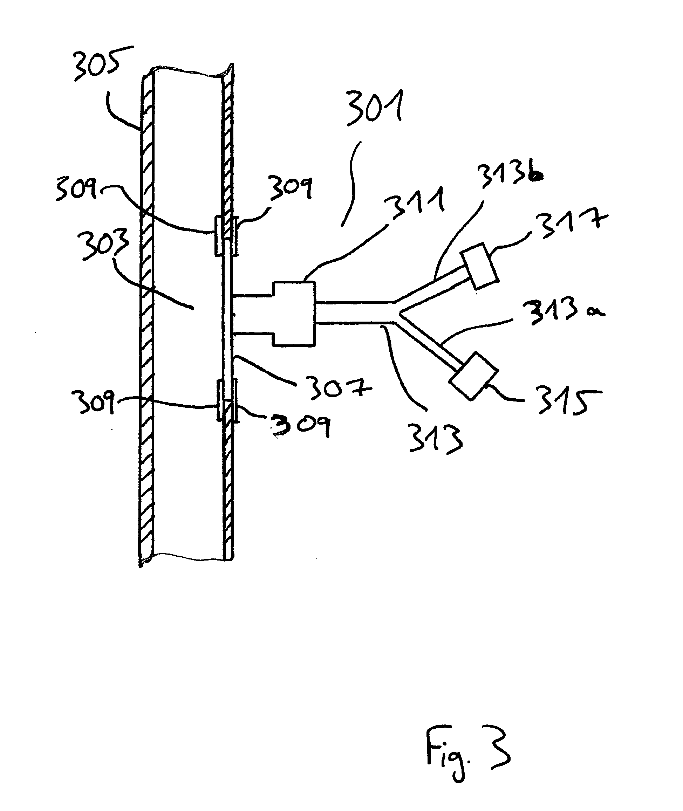

Fig. 3 zeigt einen Sensor 301. Der Sensor 301 umfasst eine Aufnahmeeinrichtung 303 , welche in diesem Ausführungsbeispiel integral mit einem Hydraulikschlauch 305 gebildet ist. Der Hydraulikschlauch 305 weist eine Aussparung auf, in welcher ein Fenster 307 aus Saphierglas mittels Halteeinrichtungen 309 befestigt ist. Die Halteeinrichtungen 309 sind paarweise an der Innenseite und der Außenseite des Hydraulikschlauchs 305 angeordnet. Die Halteeinrichtungen 309 können beispielsweise Klammern oder ein flächiger Stab sein, wobei an einer der Innenseite bzw. Außenseite des Hydraulikschlauchs 305 zugewandten Unterseite des Stabs Klebemittel aufgetragen sind.

Senkrecht zum Fenster 307 auf einer dem Hydraulikschlauch abgewandten Außenseite des Fensters 307 ist eine Adaptereinrichtung 311, an welcher einer optischen Faser 313 angekoppelt ist, mittig angeordnet. Die optische Faser 311 ist geeignet, UV-Licht zu leiten. Die optische Faser 311 weist eine Y-Form auf und wirkt somit wie ein Strahlteiler. An einem dem Fenster 307 abgewandten Ende eines ersten Armes 313a der Y-förmige Faser 313 ist eine UV-LED 315 derart angeordnet, dass mittels der UV-LED 315 emittiertes UV-Licht in den ersten Arm 313a und dann mittels der Faser 313 durch das Fenster 307 in die Aufnahmeeinrichtung 303 eingekoppelt werden kann.

Durch den Hydraulikschlauch 305 und damit auch durch die Aufnahmeeinrichtung 303 fließt ein Hydraulikfluid (nicht gezeigt), welches mittels des eingekoppelten UV-Lichts optisch angeregt wird. Das optische angeregte Hydraulikfluid wird daraufhin Lumineszenzlicht emittieren. Das Lumineszenzlicht wird mittels des Fensters 307 aus der Aufnahmeeinrichtung 303 ausgekoppelt und mittels der optischen Faser 313 durch einen zweiten Arm 313b der Y-förmigen Faser 313 in eine UV-Photodetektoreinrichtung 317 eingekoppelt.

Die gemessenen Lumineszenzspektren werden dann mittels einer Auswerteeinrichtung (nicht gezeigt) ausgewertet. Die Auswerteeinrichtung umfasst ferner eine Speichereinrichtung (nicht gezeigt), in welcher eine Korrelation zwischen Lumineszenzspektren und einer Säurezahl gespeichert ist. Beispielsweise können die Ergebnisse der in der Fig. 2 gezeigten Versuche in die Speichereinrichtung gespeichert werden.

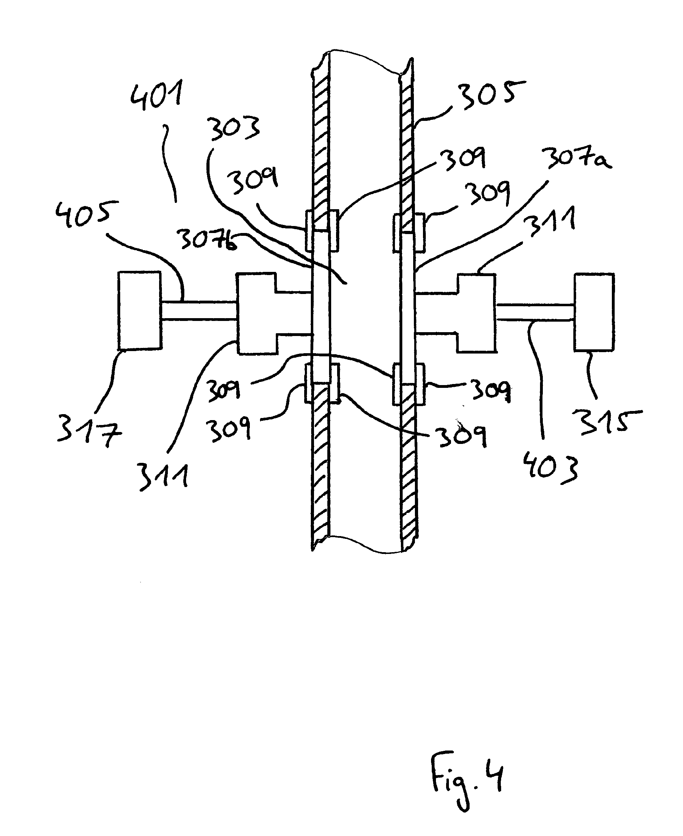

Fig. 4 zeigt einen weiteren Sensor 401, wobei für gleiche Elemente wie bei dem in Fig. 3 gezeigten Sensor die gleichen Bezugszeichen verwendet werden. Im Unterschied zu dem Sensor 301 der Fig. 3 weist der Sensor 401 zwei Fenster 307a und 307b aus Saphierglas auf, welche gegenüberliegend in jeweiligen Aussparungen des Hydraulikschlauchs 305 mittels Halteeinrichtungen 309 angeordnet sind. Analog zu Fig. 3 ist auch auf der Außenseite der Fenster 307a und 307b jeweils eine Adaptereinrichtung 311 angeordnet, an welcher jeweils eine optische Faser 403 und 405 angekoppelt ist. An den den Fenstern 307a und 307b abgewandten Enden der optischen Faser 403 und 405 ist respektive eine UV-LED 315 und UV-Photodetektoreinrichtung 317 angeordnet.

Die UV-LED 315 emittiert UV-Licht, welches mittels der Faser 403 und dem Fenster 307a in die Aufnahmeeinrichtung 303 eingekoppelt wird und dort das Hydraulikfluid optisch anregt. Das von dem optisch angeregten Hydraulikfluid emittierte Lumineszenzlicht wird dann mittels des Fenster 307b und der Faser 405 aus der Aufnahmeeinrichtung 303 ausgekoppelt und in die UV-Photodetektoreinrichtung 317 eingekoppelt. In dem in Fig. 4 gezeigten Sensor 401 sind insofern die Einkoppeleinrichtung und die Auskoppeleinrichtung derart gegenüberliegend angeordnet, dass ein optischer Strahlengang zwischen der Einkoppeleinrichtung und der Auskoppeleinrichtung gebildet ist.

### Bezugszeichenliste

- **101**: Phosphat-Ester-Molekül
- **103**: Wassermoleküle
- **105**: Alkoholgruppe
- **106**: Basisphosphatgruppe
- **107**: Phosphorsäure
- **109**: nicht-umgewandelte Phosphat-Ester-Moleküle
- **111**: desintegrierte Phosphat-Ester-Molekül
- **201, 203, 205, 207, 209**: Lumineszenzspektrum
- **301,401**: Sensor
- **303**: Aufnahmeeinrichtung
- **305**: Hydraulikschlauch
- **307, 307a, 307b**: Fenster
- **309**: Halteeinrichtung
- **311**: Adaptereinrichtung
- **313, 313a, 313b, 403, 405**: optische Faser
- **315**: UV-LED
- **317**: UV-Photodetektoreinrichtung

## Patentansprüche

1. Sensor (301) zur Online-Überwachung der Säurezahl eines Hydraulikfluids in einem Hydrauliksystem eines Luftfahrzeuges, umfassend:
- eine UV-Emittereinrichtung (315) zum Emittieren von UV-Licht,
- eine UV-Detektoreinrichtung (317) zum Detektieren von UV-Licht,
- eine Aufnahmeeinrichtung (303) zum Aufnehmen des Hydraulikfluids,
- eine Einkoppeleinrichtung (307; 313) zum Einkoppeln von mittels der UV-Emittereinrichtung (315) emittierten UV-Licht in die Aufnahmeeinrichtung (303), um das aufgenommene Hydraulikfluid optisch anzuregen, und
- eine Auskoppeleinrichtung (307; 313) zum Auskoppeln von mittels des optisch angeregeten Hydraulikfluids emittierten Licht in die UV-Detektoreinrichtung (317).

2. Sensor (301) nach Anspruch 1, wobei die Einkoppeleinrichtung (307; 313) und/oder die Auskoppeleinrichtung (307; 313) ein Fenster (307) aus einem für UV-Licht transparenten Material aufweisen.

3. Sensor (301) nach Anspruch 2, wobei das Material Saphierglas ist.

4. Sensor (301) nach einem der vorherigen Ansprüche, wobei die Einkoppeleinrichtung (307; 313) und/oder die Auskoppeleinrichtung (307; 313) eine optische Faser (313) aufweisen.

5. Sensor (301) nach einem der vorherigen Ansprüche, wobei die Einkoppeleinrichtung (307; 313) und die Auskoppeleinrichtung (307; 313) derart gegenüberliegend angeordnet sind, dass ein optischer Strahlengang zwischen der Einkoppeleinrichtung (307; 313) und der Auskoppeieinrichtung (307; 313) gebildet ist.

6. Sensor (301) nach einem der vorherigen Ansprüche, wobei die UV-Detektoreinrichtung (317) eine Auswerteeinrichtung zum Auswerten des detektierten Lichts aufweist.

7. Sensor (301) nach Anspruch 6, wobei die Auswerteeinrichtung eine Speichereinrichtung aufweist, in welcher eine Korrelation zwischen einer Wellenlänge und einer Säurezahl gespeichert ist.

8. Sensor (301) nach einem der vorherigen Ansprüche, wobei die Aufnahmeeinrichtung in dem Hydrauliksystem angeordnet ist.

9. Sensor (301) nach einem der vorherigen Ansprüche, wobei die UV-Emittereinrichtung (315) und die UV-Detektoreinrichtung (317) außerhalb des Hydrauliksystems angeordnet sind.

10. Verfahren zur Online-Überwachung der Säurezahl eines Hydraulikfluids in einem Hydrauliksystem eines Luftfahrzeuges, umfassend die folgenden Schritte:
- Emittieren von UV-Licht mittels einer UV-Emittereinrichtung (315),
- Einkoppeln des emittierten UV-Lichts mittels einer Einkoppeleinrichtung (307; 313) in eine Aufnahmeeinrichtung (303) zum Aufnehmen des Hydraulikfluids, so dass das aufgenommene Hydraulikfluid optisch angeregt wird,
- Auskoppeln von mittels des optisch angeregten Hydraulikfluids emittierten Licht mittels einer Auskoppeteinrichtung (307; 313) in eine UV-Detektoreinrichtung (317).
